# EUROPEAN PATENT APPLICATION

(11) **EP 4 104 868 A1**
(43) Date of publication of application: **21.12.2022**
(21) Application number: 22172007.1
(22) Date of filing: 06.05.2022
(51) Int. Cl.: A61L 2/10, A61L 2/24, H05B 47/115, B60Q 3/68, B60R 21/015

(54) **A DISINFECTION SYSTEM FOR A MOTORIZED VEHICLE**

(30) Priority: 17.06.2021 IN 202141027171
(71) Applicant: VOLVO TRUCK CORPORATION, 405 08 Göteborg (SE)
(72) Inventor: KANNIARAJ, Nageswaran, 610001 Tamilnadu (IN); ABUBACKKAR, Ashik, 600084 Tamilnadu (IN)
(74) Representative: Germain Maureau

(57) **Abstract**

The invention relates to a disinfection system (100) for a motorized vehicle (1) comprising:
- at least one disinfectant light emitting device (106, 108) mounted in a vehicle cabin (2) and arranged to emit disinfectant light rays towards internal surfaces of the vehicle cabin so as to sanitize them,
- first sensor means (102a-102e) adapted to sense the presence of a human or an animal inside the vehicle cabin (2) and providing a first sensing signal indicating no presence,
- a controller (104) operatively-connected to the at least one disinfectant light emitting device (106, 108) and the first sensor means (102a-102e), the controller (104) receiving said first sensing signal,
characterized in that the controller (104) is adapted to operate the at least one disinfectant light emitting device (106, 108) in response to said first sensing signal

## Description

### TECHNICAL FIELD

The invention relates to a disinfection system for a motorized vehicle.

Although the invention will be described in detail with respect to a truck, the invention is not restricted to this particular motorized vehicle, but may also be used in other motorized vehicles such as a bus, or a construction equipment or any vehicle provided with a motion system, such as an electric or thermic engine, or a hydrostatic drive.

### BACKGROUND

In the current state of the art, it is known that the application of UV light of a certain wavelength, specifically the one known as C or short wave, is an effective and environmentally friendly way to disinfect possible viruses or bacteria on all types of surfaces.

However, its effect is only positive when applied in closed places or areas.

Therefore, the application of ultraviolet light inside of vehicles to ensure their disinfection is something already known and used.

The problem is that this type of UVC light can be harmful to people's health, so it is very important that no one is inside the vehicle when it is applied.

For this reason, until now, this type of disinfection has only been applied in specialized establishments where the owner of the vehicle expressly drives his vehicle and leaves it for the time necessary to apply the treatment carried out by specialized personnel.

### SUMMARY

An object of the invention is to provide a disinfection system incorporated into the vehicle itself and permitting to the user himself to start it up when he needs it, but guaranteeing that it will always be done with all safety and without risk for people, avoiding the loss of time that involves having to leave it in a specialized workshop or establishment.

According to a first aspect of the invention, the object is achieved by a disinfection system for a motorized vehicle comprising:
- at least one disinfectant light emitting device mounted in a vehicle cabin and arranged to emit disinfectant light rays towards internal surfaces of the vehicle cabin so as to sanitize them,
- first sensor means adapted to sense the presence of a human or an animal inside the vehicle cabin and providing a first sensing signal indicating no presence,
- a controller operatively-connected to the at least one disinfectant light emitting device and the first sensor means, the controller receiving said first sensing signal, characterized in that the controller is adapted to operate the at least one disinfectant light emitting device in response to said first sensing signal.

Thus configured, the system of the present invention permits to sanitize the cabin of the vehicle without having to bring the vehicle to specific disinfection professionals and in optimal safety conditions for the user.

According to one embodiment, the at least one disinfectant light emitting device is configured to emit ultraviolet-C electromagnetic radiation.

According to a further embodiment, the at least one disinfectant light emitting device is configured to emit radiation within a wavelength range from 100 nm to 280 nm, and preferably from 250 nm to 280 nm.

According to a further embodiment, the at least one disinfectant light emitting device is configured to emit radiation having a peak intensity at 265 nm.

According to a further embodiment, the at least one disinfectant light emitting device comprises at least one light emitting diode (LED).

According to a further embodiment, the at least one disinfectant light emitting device is fixedly connected to a top wall of the vehicle cabin.

According to a further embodiment, the first sensor means comprise at least one of: a camera, a seat belt sensor adapted to sense if a seat belt is buckled, a seat sensor adapted to sense if a pressure is applied on a seat, and an ignition key sensor adapted to sense if an ignition key is positioned near or inside the vehicle cabin.

According to a further embodiment, the system further comprises second sensor means adapted to sense if the power of the vehicle is on or off and providing a second sensing signal indicating when the power is off and wherein the controller is operatively-connected to said second sensor means, the controller receiving the second sensing signal and being adapted to operate the at least one disinfectant light emitting device in response to said second sensing signal.

According to a further embodiment, the system further comprises warning means operatively-connected to the controller and adapted to provide a visible or audible alert when the at least one disinfectant light emitting device is operating.

According to a further embodiment, the warning means comprise at least one of: blinking hazard or parking lamps of the vehicle and an illuminated parking brake symbol on the dashboard of the vehicle.

According to a further embodiment, the controller is adapted to stop the operation of the warning means when the sanitizing of the vehicle cabin is completed.

According to a further embodiment, the system further comprises a human machine interface remotely connected to the controller and adapted to send an activation signal to the controller, said controller operating the at least one disinfectant light emitting device in response to the activation signal.

According to a further embodiment, the human machine interface is an ignition key provided with a specific switch, a pressure or a contact on said specific switch generating the activation signal.

According to a further embodiment, the controller is adapted to lock the vehicle doors in a closed position in response to the activation signal and to unlock the vehicle doors from said closed position when the sanitizing is completed.

The invention further relates to a vehicle comprising a disinfection system according to the invention, the vehicle being preferably a truck.

Further advantages and advantageous features of the invention are disclosed in the following description and in the dependent claims.

### BRIEF DESCRIPTION OF THE DRAWINGS

With reference to the appended drawings, below follows a more detailed description of embodiments of the invention cited as examples.

In the drawings:
Fig. 1 is a schematic side view of an example of a motorized vehicle incorporating a disinfection system according to the invention,
Fig. 2 is a schematic top view of the vehicle illustrated in Fig. 1,
Fig. 3 is a schematic front view of an example of a human machine interface that can be used in the disinfection system of the present invention, and

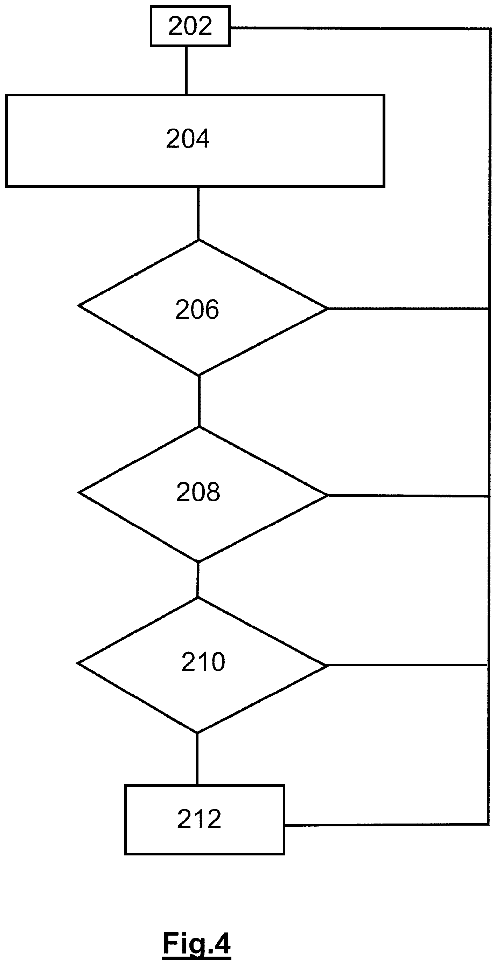

Fig. 4 is a flowchart of an example method for sanitizing the vehicle illustrated in Fig. 1 using the disinfection system according to the invention.

### DETAILED DESCRIPTION OF EXAMPLE EMBODIMENTS OF THE INVENTION

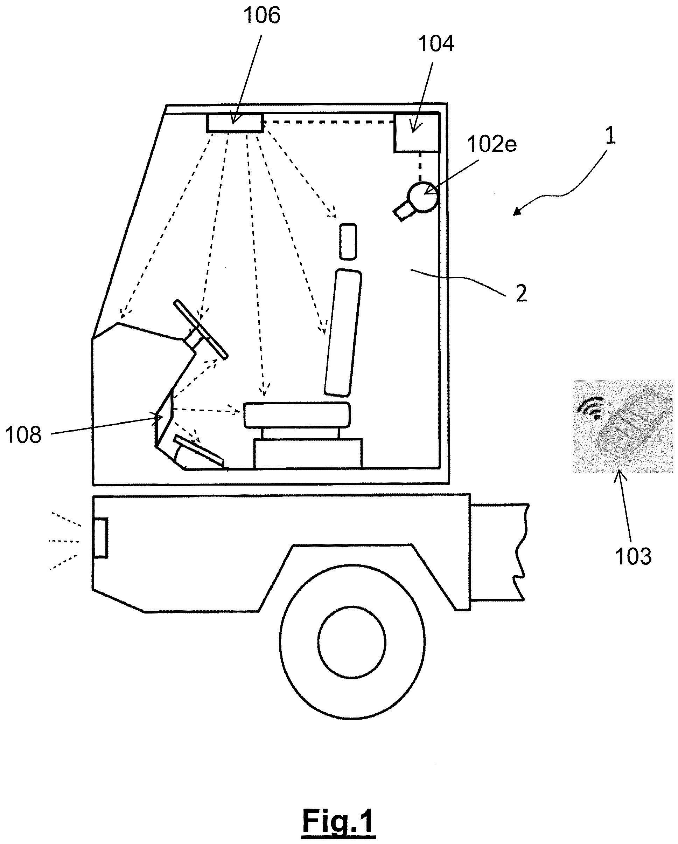

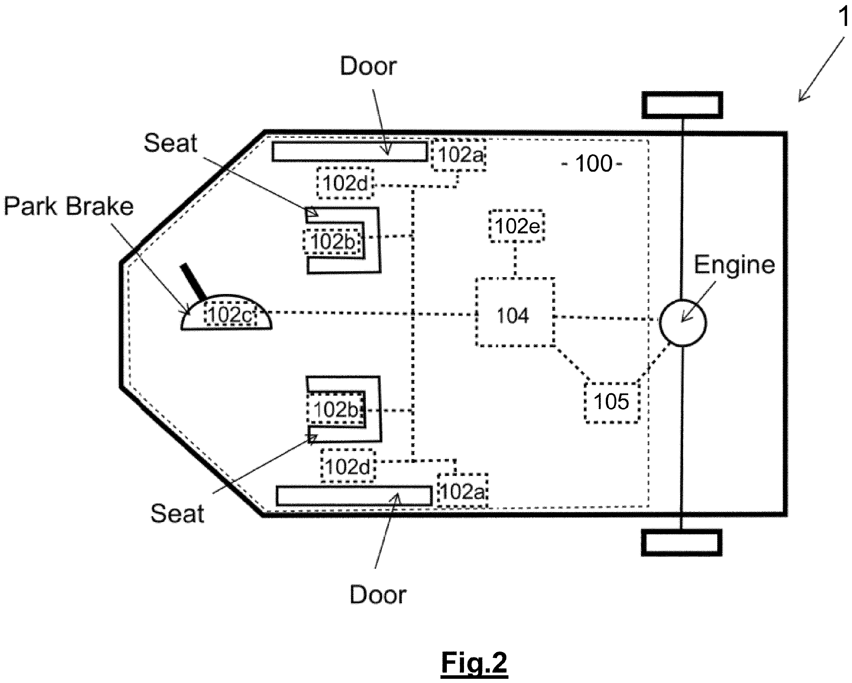

Figures 1 and 2 illustrate a truck 1 provided with a disinfection system 100 according to the present invention. The truck 1 is driven by an engine and includes a cabin 2 at the front part thereof housing the driver and the passenger(s). The cabin 3 comprises two side doors at its left and right sides, two seats, a park brake between the two seats and a seat belt for each seat.

The system 100 comprises an upper disinfectant light emitting device 106, which is fixedly connected to an upper wall of the cabin 2, and a lower disinfectant emitting device 108, which is fixedly connected to a lower wall of the cabin 2. The disinfectant emitting devices 106, 108 are constructed and arranged to shine light on and sanitize any of a number of vehicle surfaces, vehicle components or objects in the cabin 2 including, but not limited to, the dashboard, the steering wheel, the park brake, seat surfaces, door surfaces, and the pedals.

In alternative embodiments of the present invention, the system 100 may comprise only one disinfectant light emitting device or further disinfectant light emitting devices in addition to the devices 106, 108. Furthermore, the disinfectant light emitting devices may be positioned in other areas of the cabin 2 and oriented differently to sanitize other parts of the cabin 2.

In a preferred embodiment of the present invention, the disinfectant light emitting devices 106, 108 are configured to emit an ultraviolet-C (UV-C) electromagnetic radiation. Indeed, a treatment by an UV-C electromagnetic radiation minimizes microbial growth and contamination, and sanitizes vehicle surfaces. In particular, an electromagnetic radiation in the UV-C spectrum (100 nm to 280 nm), and especially within the wavelength range from 250 nm to 280 nm, is very efficient for killing germs and microorganisms, 265 nm being the peak germicidal wavelength. The intense UV-C photon energy attacks the DNA of a living cell, penetrating the cell membrane, breaking the DNA structure of the microorganism and rendering it harmless by robbing it of the ability to reproduce. An UV-C electromagnetic radiation kills bacteria, viruses and the like, even if the organisms have developed immunity to other disinfection methods.

In a preferred embodiment of the present invention, the disinfectant light emitting devices 106, 108 may comprise one or a plurality of light emitting diodes (LED) that are adapted to emit direct disinfectant light rays on the internal surfaces of the cabin 2. Such LED emitting devices are for example disclosed in the patent US 9,855,363, US 9,861,721, US 10,029,026, US 10,500,295 and US 10,814,024.

The system 100 further comprises a plurality of sensors 102a-102d located inside and/or outside of the truck 1, and adapted to sense specific operational parameters relative to specific parts of the truck 1. In particular, specific sensors 102a are adapted to sense if the doors of the truck are opened or closed. Specific sensors 102b are adapted to sense the pressure applied on the seats of the truck 1. A specific sensor 102c is adapted to sense if the park brake is applied or not. Specific sensors 102d are adapted to sense if the seat belts are buckled or not. A camera 102e is also provided to view the interior of the cabin 2. Other sensor(s) may be adapted to sense if an ignition key is positioned near or inside the vehicle cabin and also to ensure any living being is present inside the cabin (for example thermal sensor).

Furthermore, a specific sensor 105 is adapted to sense if the power is on.

The system 100 further comprises a controller 104 that is in communication with the plurality of sensors 102a-102e and 105, the controller 104 receiving sensing signals transmitted by the plurality of sensors 102a-102e and 105. The sensing signals can be analysed by the controller 104 to determine if a human or an animal is present inside the truck 1 and/or if the power is off. In particular, when the driver or a passenger leaves the truck 1, the seat belt at the driver's side, respectively the passenger's side, is not buckled, the pressure on the driver seat, respectively the passenger seat, is null, the park brake is applied and the door at the driver's side, respectively the passenger's side, is closed. Furthermore, the controller 104 may be adapted to analyse the data provided by the camera 102e to detect any motion inside the cabin 2, such a motion indicating the occupancy of the cabin 2 by a human or an animal.

The system 100 further comprises a human machine interface 103 remotely connected to the controller 104 and adapted to send an activation signal to the controller 104. In response to the activation signal, the controller 104 may operate the disinfectant light emitting devices 106, 108 depending on the other signals received from the sensors 102a-102e and 105, as explained in detail in the below paragraphs.

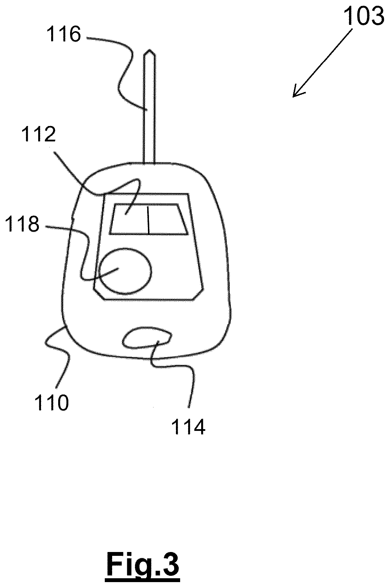

In a preferred embodiment of the present invention, the human machine interface 103 may be, for example, one or several buttons of a phone keypad, of a computer keypad or of a key used by the user to open the doors of the vehicle. As illustrated in Figure 3, the human machine interface 103 may thus be an ignition key comprising a housing 110, control buttons 112 for locking and unlocking the doors of the vehicle, an aperture 114 to pass a key ring and, at one end of the housing 110, a fixed insert 116 of elongated and substantially flat shape comprising reliefs and patterns (not shown) provided to cooperate with the lock (s) of the vehicle 1. The ignition key 103 further comprises a specific switch 118 that is adapted to generate the activation signal when a pressure or a contact is applied on said specific switch 118 by an operator.

In response to the sensing signals transmitted by the sensors 102a-102e, 105 and the activation signal transmitted by the human machine interface 103, the controller 104 is adapted to control the disinfectant light emitting devices 106, 108 to switch the system 100 between a first mode, called deactivated mode, in which no disinfectant light is emitted by the disinfectant light emitting devices 106, 108, and a second mode, called activated mode, in which the sanitizing treatment of the cabin 2 is carried out.

Figure 2 illustrates a flowchart of an example method for disinfecting the vehicle 1 illustrated in Figures 1 and 2, when the disinfecting system 100 is initially in its deactivated mode in a first step 202. At step 204, the controller 104 may receive at least one first sensing signal from the first sensors 102a-102e and a second sensing signal from the second sensor 105. In step 206, the controller 104 may determine whether a human or an animal is present inside the vehicle cabin based on the at least one second sensing signal received from the second sensors 102a-102e. For example, the controller 104 may determine that the side doors of the truck are closed based on the signal received from the sensors 102a and/or that no pressure is applied on the seats of the truck based on the signal received from the sensors 102b. If the controller 104 determines that the cabin 2 is occupied by a human or an animal, the deactivated mode is maintained and the method starts again at step 202. Otherwise, the method starts the step 208 in which the controller 104 determines if the power is off based on the second signal received from the second sensor 105. The deactivated mode is maintained if the controller 104 determines that the power is on and the method starts again at step 202. Otherwise, the next step 210 of the method consists to determine whether the activation signal has been generated by the human machine interface 103. If the controller 104 determines that the activation signal has not been generated, the deactivated mode is maintained and the method starts again at step 202. Otherwise, the system 100 is switched to its activated mode at step 212. In this activated mode, the disinfectant light emitting devices 106, 108 operate during a predetermined operating period so that the cabin 2 is entirely sanitized. The predetermined operating period may vary for example between 5 and 10 minutes. During this predetermined operating period, the controller 104 may lock the doors of the vehicle in a closed position to prevent that a human penetrates inside the vehicle and may control warning means to provide a visible or audible alert to warn that the disinfectant light emitting devices 106, 108 are operating. Such warning means may consist in the blinking hazard or parking lamps of the vehicle or in an illuminated symbol on the dashboard of the vehicle. At the end of the predetermined operating period, when the sanitizing of the vehicle cabin is completed, the controller 104 may stop the operation of disinfectant light emitting devices 106, 108, stop the operation of the warning means and unlock the doors of the vehicle from the closed position and the system 100 returns to its deactivated mode.

It is to be understood that the present invention is not limited to the embodiments described above and illustrated in the drawings; rather, the skilled person will recognize that many changes and modifications may be made within the scope of the appended claims.

## Claims

1. A disinfection system (100) for a motorized vehicle (1) comprising :
- at least one disinfectant light emitting device (106, 108) mounted in a vehicle cabin (2) and arranged to emit disinfectant light rays towards internal surfaces of the vehicle cabin so as to sanitize them,
- first sensor means (102a-102e) adapted to sense the presence of a human or an animal inside the vehicle cabin (2) and providing a first sensing signal indicating no presence,
- a controller (104) operatively-connected to the at least one disinfectant light emitting device (106, 108) and the first sensor means (102a-102e), the controller (104) receiving said first sensing signal,
**characterized in that** the controller (104) is adapted to operate the at least one disinfectant light emitting device (106, 108) in response to said first sensing signal.

2. The system (100) according to claim 1, **characterized in that** the at least one disinfectant light emitting device (106, 108) is configured to emit ultraviolet-C electromagnetic radiation.

3. The system (100) according to claim 2, **characterized in that** the at least one disinfectant light emitting device (106, 108) is configured to emit radiation within a wavelength range from 100 nm to 280 nm, and preferably from 250 nm to 280 nm.

4. The system (100) according to claim 3, **characterized in that** the at least one disinfectant light emitting device (106, 108) is configured to emit radiation having a peak intensity at 265 nm.

5. The system (100) according to any one of claims 1 to 4, **characterized in that** the at least one disinfectant light emitting device (106, 108) comprises at least one light emitting diode (LED).

6. The system (100) according to any one of claims 1 to 5, **characterized in that** the at least one disinfectant light emitting device (106, 108) is fixedly connected to a top wall of the vehicle cabin (2).

7. The system (100) according to any one of claims 1 to 6, **characterized in that** the first sensor means (102a-102e) comprise at least one of: a camera, a seat belt sensor adapted to sense if a seat belt is buckled, a seat sensor adapted to sense if a pressure is applied on a seat, and an ignition key sensor adapted to sense if an ignition key is positioned near or inside the vehicle cabin.

8. The system (100) according to any one of claims 1 to 7, further comprising second sensor means (105) adapted to sense if the power of the vehicle is on or off and providing a second sensing signal indicating when the power is off and wherein the controller (104) is operatively-connected to said second sensor means (105), the controller (104) receiving the second sensing signal and being adapted to operate the at least one disinfectant light emitting device (106, 108) in response to said second sensing signal.

9. The system (100) according to any one of claims 1 to 8, further comprising warning means operatively-connected to the controller (104) and adapted to provide a visible or audible alert when the at least one disinfectant light emitting device (106, 108) is operating.

10. The system (100) according to claim 9, **characterized in that** the warning means comprise at least one of: blinking hazard or parking lamps of the vehicle and an illuminated parking brake symbol on the dashboard of the vehicle.

11. The system (100) according to claim 9 or 10, **characterized in that** the controller (104) is adapted to stop the operation of the warning means when the sanitizing of the vehicle cabin is completed.

12. The system (100) according to any one of claims 1 to 11, further comprising a human machine interface (103) remotely connected to the controller (104) and adapted to send an activation signal to the controller (104), said controller (104) operating the at least one disinfectant light emitting device (106, 108) in response to the activation signal.

13. The system (100) according to claim 12, **characterized in that** the human machine interface (103) is an ignition key provided with a specific switch (118), a pressure or a contact on said specific switch (118) generating the activation signal.

14. The system (100) according to claim 12 or 13, **characterized in that** the controller (104) is adapted to lock the vehicle doors in a closed position in response to the activation signal and to unlock the vehicle doors from said closed position when the sanitizing is completed.

15. A vehicle (1) comprising a disinfection system (100) according to any one of claims 1 to 14, the vehicle (1) being preferably a truck.
